# EUROPÄISCHE PATENTSCHRIFT

(11) **EP 0 599 998 B1**
(45) Veröffentlichungstag und Bekanntmachung des Hinweises auf die Patenterteilung: **06.12.1995**
(21) Anmeldenummer: 92918599.9
(22) Anmeldetag: 20.08.1992
(51) Int. Cl.: A61M 15/00

(54) **VORRICHTUNG ZUM ATEMZUGSYNCHRONEN AUSLÖSEN EINES MECHANISCHEN SCHALTVORGANGES**
DEVICE FOR TRIGGERING A MECHANICAL SWITCHING PROCESS IN SYNCHRONICITY WITH BREATHING
DISPOSITIF DE DECLENCHEMENT D'UNE COMMUTATION MECANIQUE EN SYNCHRONIE AVEC LE SOUFFLE

(30) Priorität: 22.08.1991 WO PCT/EP91/01593; 08.10.1991 DE 4133274
(43) Veröffentlichungstag der Anmeldung: 08.06.1994
(73) Patentinhaber: BOEHRINGER INGELHEIM INTERNATIONAL GmbH, 55218 Ingelheim am Rhein (DE); BOEHRINGER INGELHEIM KG, 55216 Ingelheim (DE)
(72) Erfinder: KÜHNEL, Andreas, D-6370 Oberursel 6 (DE); POSS, Gerhard, D-6905 Schriesheim (DE); WITTEKIND, Jürgen, D-6000 Frankfurt 70 (DE); HOCHRAINER, Dieter, D-6530 Bingen (DE)
(74) Vertreter: Fuchs Mehler Weiss
(86) Internationale Anmeldenummer: EP9201912
(87) Internationale Veröffentlichungsnummer: WO9303783

(56) Entgegenhaltungen:
- EP-A- 0 363 060
- WO-A-90/07351
- WO-A-92/04068
- FR-A- 2 598 918
- US-A- 2 587 215
- US-A- 3 900 138
- US-A- 4 803 978

## Beschreibung

Die Erfindung bezieht sich auf eine Vorrichtung zum atemzugsynchronen Auslösen eines mechanischen Schaltvorganges in Inhalationsgeräten mit einem membranartigen Geberelement mit den Merkmalen des Oberbegriffs des Anspruchs 1, eine sie sich aus der EP-A 0 363 060 ergibt.

Inhalationsgeräte für die Verabreichung von Inhalationspräparaten im Rahmen der Atemwegstherapie sind in großer Zahl beschrieben worden. Bei solchen Geräten wird mit jeder Betätigung eine bestimmte Dosis einer Arzneimittelzubereitung freigesetzt. Je nach dem Gerätetyp kann es sich dabei um ein mikronisiertes Pulver, eine wäßrige Lösung oder eine Suspension der Arzneistoffpartikel (ggf. auch eine Lösung) in einem verflüssigten Treibgas handeln. In jedem Fall wird bei der Betätigung des Inhalationsgeräts aus der Arzneimittelzubereitung ein Aerosol erzeugt. Die Erzeugung des Aerosols muß so mit dem Atemvorgang koordiniert werden, daß eine gute Dispergierung der Teilchen in der Atemluft erfolgt und die Teilchen mit der Atemluft in die tieferen Bereiche der Lunge gelangen.

Ein Teil der Patienten, insbesondere Kinder und ältere Personen, haben häufig Schwierigkeiten, Atemzug und Erzeugung des Aerosols in geeigneter Weise aufeinander abzustimmen

Um diese Schwierigkeiten auszuschalten, sind verschiedene Lösungsvorschläge gemacht worden.

Abgesehen von der Möglichkeit, das Aerosol zunächst in ein Gefäß zu sprühen, aus dem kurz darauf eingeatmet wird (DE-A 2749629), besitzen die anderen Geräte eine Vorrichtung, die bei ausreichender Stärke des Atemzuges die Aerosolerzeugung atemzugsynchron auslöst.

Für ein mit Treibgas betriebenes Inhalationsgerät beschreibt die DE-3040641 A1 eine Sperre, die durch einen ausreichend starken Atemzug am Mundrohr des Geräts gelöst werden soll, so daß sich das Aerosol nur freisetzen läßt, wenn ein genügend starker, vom Einatmen durch das Gerät herrührender Unterdruck herrscht. Hier besteht jedoch die Gefahr, daß die Sperre festgeklemmt wird, wenn der Patient den Aerosolstoß vorzeitig auszulösen versucht.

Ein treibgasfreies Inhalationsgerät mit der eingangs bezeichneten Vorrichtung zum atemzugsynchronen Auslösen eines Aerosolstoßes ist durch die US-PS 3921637 bekannt geworden.

Bei dem bekannten Inhalationsgerät ist als - auf den Unterdruck ansprechendes - Geberelement eine mittels Feder ausbalancierte Klappe vorgesehen, die über Hebel mit einer Ventilanordnung der Schalteinrichtung im Wirkeingriff steht. Die Ventilanordnung ist in die Ausgangsleitung einer Balgenpumpe eingeschaltet, die in eine Kammer einmündet, in der Kapseln mit der zu inhalierenden Substanz eingebracht werden. Die Kammer wiederum steht mit dem Auslaß des Mundstückes in Verbindung.

Wird auf dem Pumpenbalg manuell Druck ausgeübt, und atmet der Patient am Mundstück kräftig ein, dann spricht die Klappe auf den im Mundstück entstehenden Unterdruck an, bewegt sich nach vorne und öffnet atemsynchron über die Hebel das Ventil. Der am Pumpenausgang anstehende Druck erzeugt einen Fremdluftimpuls, der die Pulverdosis aus der Kapsel unter Bildung eines Aerosols ausbläst, welches der Patient dann einatmet.

Die bekannte Vorrichtung hat wesentliche Nachteile. Die Geberanordnung ist sehr aufwendig konstruiert und wenig montagefreundlich. Sie besteht aus einer Vielzahl von sehr präzis gearbeiteten Teilen, die in einem aufwendigen Montageverfahren zusammengefügt und justiert werden müssen. Als gravierender Nachteil ist ferner zu werten, daß die Atemluft durch das Inhalationsgerät und über die mechanischen Teile der Vorrichtung strömt. Dadurch ist im Laufe der Zeit eine Staub- und Schmutzablagerung auf den präzisen Lagerstellen unvermeidbar. Dies kann insbesondere auch dadurch auftreten, daß Inhalationsgeräte dieser Art in den Kleidungstaschen der Patienten mitgeführt werden. Jede Verschmutzung der empfindlichen, mechanischen Teile der Vorrichtung kann jedoch die Funktionsfähigkeit solcher Inhalationsgeräte in Frage stellen. Vorrichtungen der eingangs genannten Art dürfen jedoch bei der Inhalation der ansaugenden Atemluft nur einen geringen Widerstand entgegensetzen. Daraus resultiert, daß für die Auslösung des Schaltvorganges, insbesondere der Erzeugung eines Fremdluftimpulses nur geringe Kräfte zur Verfügung stehen. Von allen mechanischen Teilen der Vorrichtung ist somit eine außerordentliche Leichtgängigkeit zu fordern, die im bekannten Fall nicht im notwendigen Maße gegeben ist.

Unter Hinweis darauf, daß die Auslösemechanik bei bekannten, mit vorgespannter Feder arbeitenden Mechanismen einen starken, fast ruckartigen Inspirationsfluß erforderte, schlägt die DE-C 3901963 ein mechanisch-elektronisches System vor. Dieses System erfordert indes einen relativ hohen technischen Aufwand und benötigt eine Stromquelle.

Eine gattungsgemäße Vorrichtung ist bekannt geworden aus der bereits eingangs erwähnten EP-A 0 363 060. Diese Vorrichtung weist als Geberelement eine Membran auf, die an einem Hebel in etwa in ihrer Mitte gelagert ist. Zunächst atmet der Patient Umgebungsluft ein, und zwar mit einem vorbestimmten Volumen. Danach wechselt ein Ventil seine Stellung, um die die Umgebungsluft durchlassende Bohrung zu schließen, woraufhin der Patient beim weiteren Einatmen ein in einer Kammer vorbereitetes Aerosol einatmet. Durch das vorgesehene Mundstück wird also Umgebungsluft eingeatmet, und zwar durch einen sogenannten Hauptkanal. In diesen ragt jedoch ein Venturielement, durch welches ein gewisser Luftnebenstrom aus einem von der Membran begrenzten Raum strömt. Ab einem gewissen Punkt kollabiert die Membran, worauf die Mechanik der Vorrichtung aus Hebeln und federbelastetem Stift dafür sorgt, daß das bereits erwähnte Ventil den Umgebungsluftkanal schließt.

Allerdings ist die Membran bei dieser gattungsgemäßen Vorrichtung außerhalb des Strömungsweges der Luft beim Einatmen eingebaut. Hieraus folgt per se eine gewisse Schwergängigkeit und eine nicht ganz zuverlässige Funktion, beispielsweise durch das Verschieben des Schaltpunktes durch Aerosolreste etc..

Ein weiteres Inhalationsgerät ist bekannt aus der DE-A 19 45 257.

Dieses Gerät weist eine Vorrichtung zum Auslösen eines Schaltvorganges auf, die sich einer an ihrem Rande eingespannte Membran bedient. Bei dieser Vorrichtung ist die Membran allerdings nicht außerhalb des Strömungsweges der Luft beim Einatmen eingebaut, wodurch sich einige Komplikationen ergeben können, beispielsweise durch eine vom Patienten aufzubringende relativ hohe Ansaugkraft, um den Inhalationsvorgang auszulösen. Gerade dies ist aber von Patienten mit atemwegsbedingten Schwierigkeiten nicht von vorn herein zu erwarten.

Vor diesem Hintergrund liegt der Erfindung die Aufgabe zugrunde, ausgehend von dem gattungsbildenden Stand der Technik eine Vorrichtung zum atemzugsynchronen Auslösen von Schaltvorgängen bei Inhalationsgeräten zu schaffen, die einfach gebaut und leichtgängig ist, keine Stromquelle erfordert, konstruktionsbedingt nicht zum Verschmutzen neigt und deshalb über lange Zeit verläßlich ihre Funktion erfüllt, auch wenn der Schaltvorgang, weil rein mechanisch, schwieriger als im elektronischen Fall auszulösen ist.

Die Lösung dieser Aufgabe gelingt gemäß der Erfindung dadurch, daß ein Membranraum vorgesehen ist, der auf der einen Seite durch die Membran begrenzt ist und der einen Membrantopf aufnimmt, dem Auslösestifte zugeordnet sind, die beim Ansprechen der Membran auf den Unterdruck mit dem Schalt- und Auslösemechanismus an der Schalteinrichtung in Wirkverbindung gebracht werden.

Gemäß einer Weiterbildung der Erfindung besitzt das Inhalationsgerät ein Mundstück mit einem Luftkanal, der als Düse ausgebildet ist, wobei die Verbindung zwischen Membran und Mundstück durch einen Unterdruckkanal hergestellt wird, dessen Einmündung in das Mundstück im Bereich der höchsten Strömungsgeschwindigkeit angeordnet ist.

Dadurch wird ein besonders hoher Unterdruck erzeugt, der einen sicheren Schaltvorgang bedingt.

Gemäß einer vorteilhaften Weiterbildung ist vorgesehen, daß die Schalteinrichtung eine Pumpe zur Erzeugung eines atemzugsynchronen Fremdluftimpulses triggert, die mit einer entriegelbaren Vorspanneinrichtung 13) in Verbindung mit einem Auslösemechanismus für die Entriegelung der Vorspannung, der im Wirkeingriff mit den mechanischen Auslöseelementen steht, versehen ist.

Eine derartige Vorrichtung ist vielseitig einsetzbar. Mit dem Fremdluftimpuls kann beispielsweise eine zu inhalierende Dosis einer pulverförmigen medizinischen Substanz atemzugsynchron dispergiert werden, etwa die auf einem bandförmigen Träger gemäß WO 90/13328 bereitgestellt Dosis eines Inhalationspulvers.

Mit dem Fremdluftimpuls kann auch eine Flüssigkeit dispergiert werden.

Weitere Ausgestaltungen und Anwendungsmöglichkeiten der Erfindung ergeben sich anhand von in den Zeichnungen beschriebenen Ausführungsbeispielen. Als Anwendungsbeispiel ist ein Inhalationsgerät mit einer Dosiervorrichtung und einer durch den Membrangeber triggerbaren Pumpe als Fremdluftimpulsgeber gezeigt, jedoch ist die Erfindung nicht auf solche Anwendungen beschränkt.

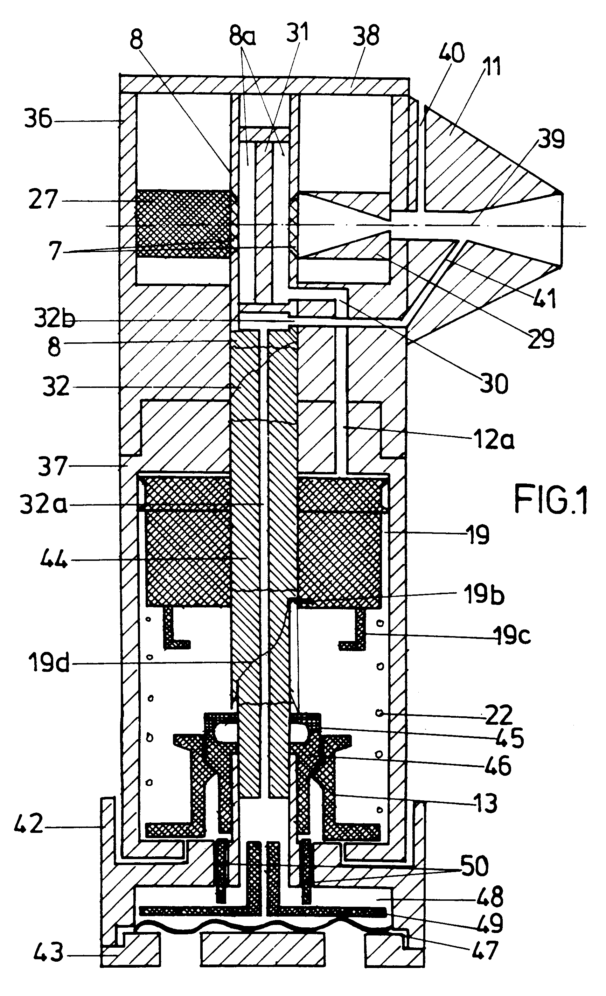

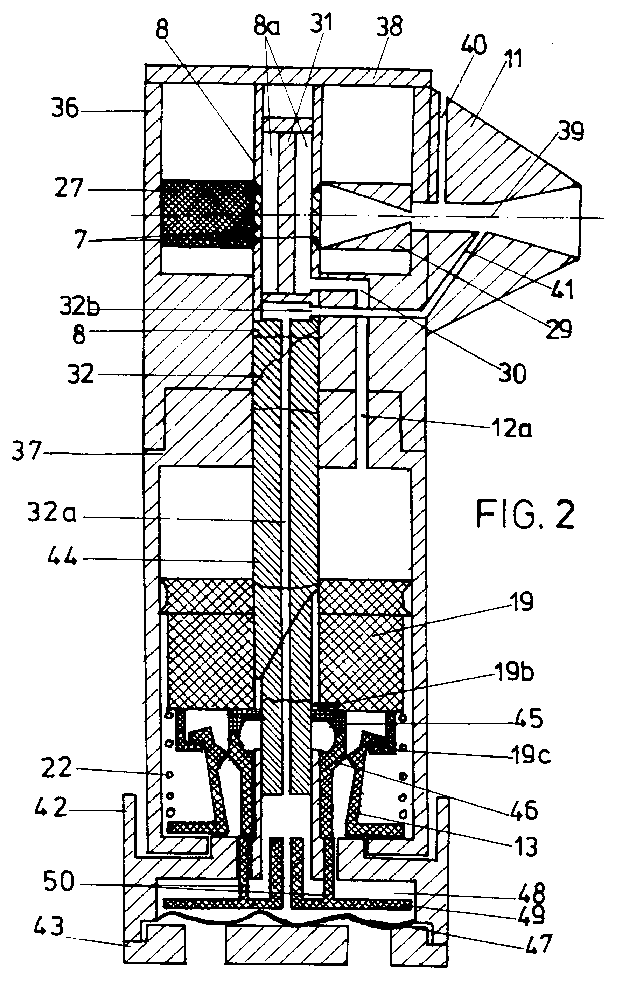

Es zeigen:
- Fig. 1:: ein Ausführungsbeispiel eines treibgasfreien Inhalationsgerätes im Normalzustand in einer geschnittenen Prinzip-Ansicht mit der erfindungsgemäßen Vorrichtung;
- Fig. 2:: die Ausführungsform nach Figur 1 in der Bereitschaftsstellung zum Inhalieren,
Das Inhalationsgerät nach den Figuren 1 und 2 weist ein zweiteiliges Gehäuse auf, nämlich das Kopfteilgehäuse 36, das oben mit einem Deckel 38 abgeschlossen ist, und das Pumpengehäuse 37, das gleichzeitig den Zylinder für den Kolben 19 der Pumpanordnung bildet. Beide Gehäuseteile bestehen vorzugsweise aus Kunststoff und weisen übliche nicht dargestellte Verbindungselemente, z.B. eine Schraubverbindung auf.

Im Kopfteilgehäuse 36 ist eine Dosiervorrichtung mit einer Pulverschüttung 27 untergebracht. Sie weist ein drehbares Teil 8 mit zwei Dosierkerben 7 auf, denen jeweils eine Kammer 8a zugeordnet ist, die, getrennt durch eine Wandung 31, alternierend mit einem Kanal 30 verbindbar sind, derart, daß stets nur die "auszublasende" Dosierkerbe über die zugeordnete Kammer mit dem dispergierenden Druckluftstoß aus der noch zu erläuternden triggerbaren Pumpe anströmbar ist. Im unteren Teil des Teils 8 befindet sich ein Kupplungsteil 32, über den das Teil 8 mit einem Drehantrieb verbindbar ist. Dieser Abschnitt des Teils 8 weist eine Bohrung 32b für einen Unterdruckkanal und eine Längsbohrung 32a zur Weiterleitung des Unterdruckes auf, deren Bedeutung noch erläutert wird.

Mit der auszublasenden Dosierkerbe 7 bzw. der zugeordneten Kammer im Teil 8 steht eine Dispergierdüse 29 in Verbindung.

Am Kopfteilgehäuse ist ferner ein Mundstück 11 angebracht, das der Dispergierdüse 29 gegenüber liegt. Das Mundstück 11 besitzt einen Luftkanal in Form einer Inhalationsdüse 39 sowie Atemluftbohrungen 40 für die nachströmende Außenluft beim Inhalieren. Das Mundstück 11 weist ferner einen Unterdruckkanal 41 auf, der mit der Bohrung 32b am Teil 8, und damit mit der Längsbohrung 32a in Verbindung steht. Beim aktiven Einatmen entsteht infolge der Beschleunigung, die die Luft in der Düse erfährt, in der Düse und damit im Kanal 41 ein Unterdruck, der sich in die Längsbohrung 32a fortsetzt. Der Unterdruckkanal befindet sich daher zweckmäßig an der Stelle in der Düse, in der die höchste Geschwindigkeit herrscht.

Das Pumpengehäuse 37 weist einen Drehknopf 42 auf, der mit einer Spannwelle 44 verbunden und stirnseitig durch einen mit Bohrungen versehenen Deckel 43 abgeschlossen ist. Die Spannwelle 44 weist, wie das Teil 8, mit dem sie über die Kupplung 32 positionsgenau und torsionskraftschlüssig verbindbar ist, die Axialbohrung 32a auf.

In dem Pumpengehäuse 37 ist im vorderen Teil eine spann- und triggerbare Kolbenpumpe untergebracht. Die Pumpe weist den Pumpenkolben 19 auf, der einen Stift 19b besitzt, der in einer Spiralnut 19d der Spannwelle 44 geführt ist. In kinematischer Umkehr dieses Prinzips der translatorischen Umsetzung einer Drehbewegung können an der Spannwelle Nocken oder dergleichen vorgesehen sein, die in einer Spiralnut aufgenommen sind, die sich in der Kolbenbohrung befindet.

In Figur 1 befindet sich der Kolben in der oberen Stellung nach der Triggerung und dem Luftausstoß, wogegen die Figur 2 den gespannten Kolben zeigt. In dieser Darstellung ist besonders gut der Pumpzylinderraum oberhalb des Kolbens 19, in dem die Luft durch den hochgehenden Kolben komprimiert wird, zu erkennen. An dem Pumpzylinderraum befindet sich die Luftaustrittsöffnung, der Druckkanal 12 a, der in die Bohrung 30 am Teil 8 zwecks Weiterleitung der dispergierenden Druckluft in die jeweilige Kammer 8a bzw. Dosierkerbe 7 einmündet.

An der unteren Kolbenseite ist ein radialsymetrisches Klinkelement 19c, eine Halteklammer vorgesehen, mit dem der Kolben gegen die Kraft der Feder 22 unter Eingriff mit einer ebenfalls radialsymmetrischen Verriegelungsklammer 13, die elastisch nachgebende Segmente besitzt, vorspannbar ist.

Die aufeinanderliegenden Teile der Halte- und Verriegelungsklammer weisen eine leichte Schräge auf, derart, daß die Halteklammer 19c unter dem Einfluß der Kraft der Feder 22 das Bestreben hat, die Segmente der Verriegelungsklammer nach innen zu bringen und damit die Verriegelung zu öffnen. Die Schräge unterstützt daher die Auslösung, zusätzlich unterstützt durch die Eigenspannung der Segmente der Verriegelungsklammer. Der Verklink- und Auslösemechanismus weist ferner einen Rückstellknopf 45, der sich mit der Spannwelle 44 mitdreht, sowie einen Auslöseknopf 46 mit einer Verriegelungsschulter, der längsaxial verschiebbar ist, auf. Beim Spannen des Kolbens wird der Auslöseknopf 46 über den Rückstellknopf 45 oberhalb seiner Verriegelungsschulter in die Verriegelungs- und zugleich Auslöseklammer 13 gedrückt, derart, daß die Auslösekante der Auslöseklammer 13 oberhalb der Verriegelungsschulter des Auslöseknopfes 46 liegt. Dabei rastet die Verriegelungs- und Auslöseklammer in das Klinkelement oder Haltklammer 19c des Kolbens ein (Figur 2).

Die aufeinandergleitenden, mit einer Kulisse versehenen Kanten von Rückstell- und Auslöseknopf 45 und 46 sind rampenartig gestaltet. Im gespannten Zustand (Figur 2) ist der höchste Punkt der Rampe bereits überschritten, so daß der Raum hinter dem Rampenabbruch für die notwendige axiale Bewegung des Auslöseknopfes beim Auslösen zur Verfügung steht.

Die dargestellte Verklinkung stellt eine verhältnismäßig einfache Lösung dar, die auch maschinell leicht montierbar ist.

Zum selbsttätigen Auslösen der Pumpe beim aktiven Einatmen ist eine Ausführung der erfindungsgemäßen Triggermechanik vorgesehen, die als zentrales Element eine Membran 47 aufweist, die auf den beim Einatmen entstehenden Unterdruck im Unterdruckkanal 41 bzw. fortpflanzend in der Axialbohrung 32a, anspricht. Die Membrane 47 begrenzt stirnseitig einen Membranraum 48, in dem sich ein Membrantopf 49 befindet, an dem Auslösestifte 50 anliegen, die im Drehknopf 42 geführt sind. Mit ihrem anderen Ende liegen diese Auslösestifte am Auslöseknopf 46 an.

Für die Einstellung des inhalationsbereiten, gespannten Zustandes gemäß Figur 2 aus dem ungespannten Ruhezustand nach Figur 1 sind folgende Handlungen vorzunehmen:
Der Drehknopf 42 wird manuell um einen bestimmten Winkel verdreht. Mit dem Drehknopf drehen sich mit die Spannwelle 44, der Rückstellknopf 45 und gleichzeitig das Teil 8 (über die Kupplung 32). Im Ausführungsbeispiel ist der Steigungswinkel der Spiral-Nut 19d so gewählt, daß eine Verdrehung von 180° notwendig ist, um den Spannzustand zu erreichen.

Durch die Drehung des Drehknopfes 42 wird zunächst über die Drehung des Teils 8 im Kopfteilgehäuse 36 die Dosierkerbe 7 mit dem zu inhalierenden Pulver befüllt. Weiterhin wird durch das Drehen der Spannwelle 44 der in der Spiralnut 19d der Spannwelle geführte Stift 19b des Kolbens 19 nach unten bewegt. Dabei spannt der Kolben 19 die Feder 22. Nach einem Drehwinkel von ca. 135° befindet sich der Kolben an sich in der Ausgangsstellung. Beim Drehen des Knopfes 42 um 45° wird der Auslöseknopf 46 über den Rückstellknopf 45 mit seiner Verriegelungsschulter in die Auslöseklammer 13 gedrückt. Dabei rastet diese Klammer in das Klinkelement 19c des Kolbens kraftschlüssig ein.

Durch diese Ausgestaltung ist das Gerät mit einer einzigen Spannbewegung hinsichtlich der Dosierung und der Atemlufttriggerung initialisierbar.

Der Kolben ist jetzt gespannt und wird durch die beschriebene Verriegelung in seiner Position gehalten. Die Schrägen an der Verriegelungsklammer 13 und an dem Klinkelement 19c sind so ausgeführt, daß das Klinkelement unter dem Einfluß der Federkraft das Bestreben hat, die Verriegelungsklammer nach innen zu bringen und damit die Verriegelung zu öffnen. Dies wird jedoch durch den Auslöseknopf 46 verhindert, dessen dickeres Oberteil gegen die Nocken der Verriegelungsklammer drückt und diese im gespreizten Zustand hält. Dadurch ergibt sich eine besonders vorteilhafte Sicherheit gegen unbeabsichtigtes Fehlauslösen des Gerätes.

Das Inhalationsgerät befindet sich nun im Bereitschaftszustand nach Figur 2, d.h. ist bereit zum Inhalieren.

Bei der Inhalation wird beim Einatmen durch das Mundstück 11 über die Fremdluftöffnung 40 Luft zugeführt. Durch diese an der Bohrung 41 und der Düse 39 vorbeiströmende Umgebungsluft entsteht ein Unterdruck in dieser Bohrung 41, der über die Axialbohrung 32a bis in den Membranraum 48 weitergeführt wird. Durch den atmosphärischen Druck, der über die Bohrungen im Deckel 43 anliegt, wird die Membran 47 nach innen auf den Membrantopf 49 gedrückt. Dieser drückt auf die Auslösestifte 50, die ihrerseits am Auslöseknopf 46 anliegen und bei Erreichen eines bestimmten Unterdruckes auslösen, indem die Verriegelungsschulter des Auslöseknopfes 46 durch eine Axialbewegung dieses Knopfes über die Auslösekante (Nocke) der Verriegelungsklammer 13 gebracht wird. Die Nocken der Verriegelungsklammer 13 gelangen dabei in den Bereich des dünneren Schaftes des Auslöseknopfes und können sich nicht mehr an diesem abstützen. Unter dem Einfluß der Eigenspannung der Segmente der Verriegelungsklammer, die bestrebt ist die Federsegmente nach innen zu biegen, und unter dem Einfluß der an den Schrägen der Verriegelungsklammer und des Klinkelementes 19c auftretenden, nach innen gerichteten Kraft biegen sich die Segmente der Verriegelungsklammer nach innen und der Kraftschluß zwischen der Klammer und dem Klinkelement 19c wird aufgehoben.

Durch diese doppelt zur Entriegelung wirkenden Kräfte wird vorteilhafterweise eine besonders hohe Auslösesicherheit bewirkt.

Der Kolben 19 wird durch die Kraft der Feder 22 nach oben bewegt. Der entstehende Luftdruckstoß wird durch den Druckkanal 12a weitergeleitet und gelangt über die Bohrung 30 in die rechte Kammer 8a der Dosierungsvorrichtung. Das in der (rechten) Dosierkerbe 7 befindliche Pulver wird über die Düse 29 dispergiert und dem Atemluftstrom beigemischt, d.h. in ein Aerosol überführt. Danach befindet sich das Gerät wieder im Ausgangszustand nach Figur 1.

Die in den Figuren 1 und 2 verwendeten Baugruppen und -elemente sind Ausführungsformen; die Erfindung ist darauf jedoch nicht beschränkt. So können beispielsweise auch andere Konstruktionselemente zum Umsetzen einer Drehbewegung in eine Längsverschiebung des Kolbens 19 bzw. andere Verklink- und Auslösemechanismen verwendet werden, ohne daß die Erfindung deswegen verlassen würde.

Anhand der Figur 1 und 2 werden die Vorteile der Vorrichtung nochmals deutlich.

Die Atemluft bzw. die Fremdluft hat im zugehörigen Inhalationsgerät einen sehr kurzen Weg zum Mundstück. Die Fremdluft durchströmt vorher nur den Dosierstift und die Dosierkammer. Eine Staub- und Schmutzablagerung wird daher vermieden, die Teile bleiben leichtgängig. Bei einer dringend erforderlichen Applikation des Aerosols, etwa bei einem akuten Asthmaanfall, ist somit eine außerordentlich einfache und schnelle Handhabung des Gerätes möglich.

Die Teile der Konstruktion sind ferner verhältnismäßig einfach und auch leicht zu montieren, damit ist das Gerät kostengünstig in großer Stückzahl herstellbar.

Durch geeignete Modifizierung, wie sie der Fachmann ohne weiteres vornehmen kann, läßt sich die erfindungsgemäße Vorrichtung so adaptieren, daß sie mit den verschiedensten Gerätetypen zusammenwirkt, die eine atemzuggetriggerte bzw. atemzugssynchrone Auslösung vorsehen. Somit bietet sich die Anwendung der neuen Vorrichtung z.B. in Geräten an, wie sie in der DE-A 1917911, DE-A 1945257, DE-A 3040641, DE-C 3901961, WO 90/13327, WO 90/13328, PCT(GB91/00433, US-A 3921637, US-A 3187748 oder US-A 4648393 beschrieben sind, ohne daß die Anwendung der Erfindung auf diese Gerätetypen beschränkt wäre.

## Patentansprüche

1. Vorrichtung zum atemzugsynchronen Auslösen eines mechanischen Schaltvorganges in Inhalationsgeräten mit einem Geberelement, welches auf den Unterdruck beim Einatmen durch das Inhalationsgerät anspricht, und einer Schalteinrichtung, die mit dem Geberelement in Wirkeingriff steht, wobei das Geberelement eine flexible, am Rande eingespannte Membran (47) ist, die außerhalb des Strömungsweges des Hauptluftstromes beim Einatmen so in das Inhalationsgerät eingebaut ist, daß ihre eine Seite mit der Umgebungsluft in Verbindung steht und auf ihre andere Seite der beim Einatmen durch das Inhalationsgerät entstehende Unterdruck wirkt und der mechanische Auslöseelemente (13, 45, 46, 50) zugeordnet sind, die mit dem Auslösemechanismus an der Schalteinrichtung im Wirkeingriff stehen,
dadurch gekennzeichnet,
daß ein Membranraum (48) vorgesehen ist, der auf der einen Seite durch die Membran (47) begrenzt ist und der einen Membrantopf (49) aufnimmt, dem Auslösestifte (50) zugeordnet sind, die beim Ansprechen der Membran auf den Unterdruck mit dem Schalt- und Auslösemechanismus (13, 19c, 45, 46) an der Schalteinrichtung in Wirkverbindung gebracht werden.

2. Vorrichtung nach Anspruch 1, dadurch gekennzeichnet, daß die Schalteinrichtung eine Pumpe (19) zur Erzeugung eines atemzugsynchronen Fremdluftimpulses triggert, die mit einer entriegelbaren Vorspanneinrichtung (22, 19c, 13) in Verbindung mit einem Auslösemechanismus (45, 46) für die Entriegelung der Vorspannung, der im Wirkeingriff mit den mechanischen Auslöseelementen (49, 50) steht, versehen ist.

3. Vorrichtung nach Anspruch 2, dadurch gekennzeichnet, daß die Pumpe einen Pumpenraum mit einem Luftaustrittskanal (12a) und einem Pumpenkolben (19), welcher mittels manueller Betatigung gegen die Kraft einer Feder (22) vorspannbar und auf der dem Luftaustrittskanal (12a) abgewendeten Seite des Pumpenraumes verklinkbar ist, aufweist.

4. Vorrichtung nach Anspruch 3, dadurch gekennzeichnet, daß zur auslösbaren Verklinkung an der dem Pumpenraum abgewandten Kolbenseite eine radialsymmetrische Halteklammer (19c) angebracht ist, die eine leicht schräge Haltefläche aufweist, die beim Spannen des Kolbens (19) mittels eines Auslöseknopfes (46) in Wirkverbindung mit einer ebenfalls leicht schrägen Fläche einer radialsymmetrischen Verriegelungsklammer (13) bringbar ist, die elastisch nachgebende Segmente besitzt, welche durch den Auslöseknopf (46) mit den Auslöseelementen (50) der auf den Unterdruck ansprechenden Membran (47) in Wirkverbindung ist, derart, daß beim atemzugsynchronen Ansprechen der Membran der Auslöseknopf (46) zumindest teilweise unter Aufhebung der Spreizung der Segmente der Verriegelungsklammer und damit der Verklinkung aus der Verriegelungsklammer (13) austreibbar ist.

5. Vorrichtung nach Anspruch 4, dadurch gekennzeichnet, daß der Auslöseknopf (46) eine radiale Verdickung mit Schulteransatz und die Verriegelungsklammer (13) eine umlaufende Kante oder Nocken aufweist, derart, daß im vorgespannten Zustand des Kolbens (19) die Kante bzw. Nocken an der Verdickung anliegen.

6. Vorrichtung nach einem der Ansprüche 1 bis 5, mit einem Gehäuse (36, 37) für das Inhalationsgerät, an welchem an einem Ende seitlich ein Mundstück (11) befestigt ist, wobei in diesem Gehäuseteil eine Dosiereinrichtung (8) für die zu inhalierende Wirksubstanz untergebracht ist, dadurch gekennzeichnet, daß im anderen Ende des Gehäuses die auf Unterdruck ansprechende Membran (47) einschließlich des Auslösemechanismus für die vorgespannte Pumpe aufgenommen ist und im Mittelteil des Gehäuses die triggerbare Pumpe angeordnet ist.

7. Vorrichtung nach Anspruch 6, dadurch gekennzeichnet, daß von dem Mundstück (11) durch das Inhalationsgerät hindurch bis hin zur Membran ein Unterdruckkanal (41, 32a) geführt ist.

## Claims

1. Device for initiating a mechanical switching operation, in synchronism with the breathing, in inhalers having a transmitter element which responds to the low pressure as the user breathes in through the inhaler, and a switching device which is operatively connected to the transmitter element, the transmitter element being a flexible membrane (47) clamped around its edges which is incorporated in the inhaler, outside the flow path of the main air current as the user breathes in, so that one side of said membrane is connected to the ambient air whilst the other side thereof is acted upon by the low pressure produced as the user breathes in through the inhaler, and which has associated mechanical actuating elements (13,45,46,50) which are operatively connected to the actuating mechanism on the switching device, characterised in that a membrane chamber (48) is provided, which is bounded on one side by the membrane (47) and which accommodates a membrane pot (49) which is associated with actuating pins (50) which can be operatively connected to the switching and actuating mechanism (13,19c,45,46) on the switching device when the membrane responds to the low pressure.

2. Device according to claim 1, characterised in that the switching device triggers a pump (19) for generating a pulse of foreign air in synchronism with the breathing, this pump being provided with releasable biasing means (22,19c,13) connected to an actuating mechanism (46,46) for releasing the bias, said actuating mechanism being operatively connected to the mechanical actuating elements (49,50).

3. Device according to claim 2, characterised in that the pump has a pump chamber with an air outlet channel (12a) and a pump piston (19) which can be biased by manual actuation counter to the force of a spring (22) and can be latched on the side of the pump chamber which is remote from the air outlet channel (12a).

4. Device according to claim 3, characterised in that, for the releasable latching, a radially symmetrical retaining bracket (19c) is mounted on the end of the piston remote from the pump chamber, this retaining bracket having a slightly inclined retaining surface which, when the piston (19) is tensioned by means of an actuating knob (46), can be brought into operative connection with an equally slightly inclined surface of a radially symmetrical locking bracket (13) having resiliently yielding segments, which is effectively connected with the actuating elements (50) of the membrane (47) responding to the low pressure, by means of the actuating knob (46), so that when the membrane responds in synchronism with the breathing the actuating knob (46) can be at least partially pushed out of the locking bracket (13), thereby reversing the spreading of the segments of the locking bracket and hence the latching action.

5. Device according to claim 4, characterised in that the release knob (46) has a radially thickened portion with shoulder projection and the locking bracket (13) has an encircling edge or cam so that in the biased condition of the piston (19) the edge or cam abuts on the thickened portion.

6. Device according to one of claims 1 to 5, having a housing (36,37) for the inhaler on which a mouth piece (11) is secured laterally at one end, whilst in this housing portion is accommodated a metering device (8) for the active substance which is to be inhaled, characterised in that, in the other end of the housing, the membrane (47) which responds to low pressure, including the release mechanism for the biased pump, is accommodated and the actuable pump is arranged in the centre part of the housing.

7. Device according to claim 6, characterised in that a low pressure channel (41,32a) leads from the mouth piece (11) through the inhaler and as far as the membrane.

## Revendications

1. Dispositif de déclenchement en synchronisme avec le souffle d'une opération de commutation mécanique, dans des inhalateurs, comportant un capteur, qui réagit à la dépression apparaissant lors de l'inhalation à travers l'inhalateur, et un dispositif de commutation, qui est en liaison active avec le capteur, le capteur étant une membrane (47) flexible, tendue sur son bord, qui est installée dans l'inhalateur en-dehors du chemin d'écoulement du flux d'air principal, lors de l'installation, de telle sorte que son premier côté soit en contact avec l'air ambiant alors que son autre côté est exposé à la dépression, qui apparaît lors de l'inhalation à travers l'inhalateur et à laquelle sont associés des éléments (13, 45, 46, 50) de déclenchement mécaniques qui sont en liaison active avec le mécanisme de déclenchement au niveau du dispositif de commutation,
caractérisé en ce que,
un espace de membrane (48) est prévu, qui est délimité du premier côté par la membrane (47) et qui reçoit une coupelle de membrane (49), à laquelle sont associées des broches de déclenchement (50), qui, lorsque la membrane réagit à la dépression, sont amenées en liaison active avec le mécanisme de commutation et de déclenchement (13, 19c, 45, 46) au niveau du dispositif de commutation.

2. Dispositif suivant la revendication 1, caractérisé en ce que le dispositif de commutation déclenche une pompe (19) afin de produire une impulsion d'air extérieur en synchronisme avec le souffle, la pompe étant pourvue d'un dispositif de mise sous prétension déverrouillage (22, 19c, 13) en liaison avec un mécanisme de déclenchement (45, 46) pour le déverrouillage de l'état de prétension, qui est en liaison active avec les éléments (49, 50) de déclenchement mécaniques.

3. Dispositif suivant la revendication 2, caractérisé en ce que la pompe comporte une chambre avec un canal de sortie d'air (12a) et un piston (19), qui peut être mis sous prétension manuellement à l'encontre de la sollicitation d'un ressort (22) et qui peut être encliqueté du côté de la chambre de pompe opposé au canal de sortie d'air (12a).

4. Dispositif suivant la revendication 3, caractérisé en ce que, pour l'encliquetage déclenchable, un crochet de retenue (19c) à symétrie radiale est prévu sur le côté du piston opposé à la chambre de pompe, lequel crochet de retenue présente une surface de retenue légèrement inclinée, qui lors de la mise sous tension du piston (19) peut être amenée au moyen d'un bouton de déclenchement (46) en liaison active avec une surface également légèrement inclinée d'un arrêtoir de verrouillage (13) à symétrie radiale, qui comprend des segments élastiques, qui sont en liaison active par l'intermédiaire du bouton de déclenchement (46) avec les éléments de déclenchement (50) de la membrane (47) réagissant à la dépression, de telle sorte que, lors de la réaction de la membrane en synchronisme avec le souffle, le bouton de déclenchement (46) peut être retiré, du moins en partie, du fait de la suppression de l'écartement des segments de l'arrêtoir de verrouillage et donc de l'encliquetage, hors de l'arrêtoir de verrouillage (13).

5. Dispositif suivant la revendication 4, caractérisé en ce que le bouton de déclenchement (46) présente un épaississement radial avec des épaulements et l'arrêtoir de verrouillage (13) présente un bord périphérique ou une saillie, de telle sorte que, dans l'état sous prétension du piston (19) le bord ou la saillie s'appuie sur l'épaississement.

6. Dispositif suivant l'une quelconque des revendications 1 à 5, comportant un boîtier (36, 37) pour l'inhalateur, auquel est fixée à une extrémité, latéralement, une embouchure (11), un dispositif de dosage (8) pour la substance active à inhaler étant placé dans cette partie du boitier, caractérisé en ce que dans l'autre extrémité du boîtier, est placée la membrane (47), réagissant à la dépression, y compris le mécanisme de déclenchement de la pompe mise sous prétension et dans la partie centrale du boitier, la pompe déclenchable.

7. Dispositif suivant la revendication 6, caractérisé en ce qu'un canal à dépression (41, 32a) part de l'embouchure (11) et traverse l'inhalateur jusqu'à la membrane.
